Europäisches Patentamt

European Patent Office

Office européen des brevets

(19)

(11) **EP 0 756 820 B1**

(12) **EUROPÄISCHE PATENTSCHRIFT**

(45) Veröffentlichungstag und Bekanntmachung des Hinweises auf die Patenterteilung:
**21.03.2001 Patentblatt 2001/12**

(51) Int Cl.$^7$: **A01N 25/12**, A01N 25/10, A01N 59/12

(21) Anmeldenummer: **96111587.0**

(22) Anmeldetag: **18.07.1996**

(54) **Zubereitungen, enthaltend ein Iodophor aus Poly-N-vinyllactam und Dextrin und Verwendung solcher Zubereitungen**

Compositions comprising an iodophor of poly-N-vinyllactam and dextrin and use thereof

Compositions contenant un iodophor à base de lactame polyvinylique et dextrin et leur utilisation

(84) Benannte Vertragsstaaten:
**AT BE CH DE DK ES FR GB IT LI NL SE**

(30) Priorität: **31.07.1995 DE 19527714**

(43) Veröffentlichungstag der Anmeldung:
**05.02.1997 Patentblatt 1997/06**

(73) Patentinhaber: **BASF Aktiengesellschaft**
**67063 Ludwigshafen (DE)**

(72) Erfinder:
• **Breitenbach, Jörg, Dr.**
**68199 Mannhein (DE)**

• **Sanner, Axel, Dr.**
**67227 Frankenthal (DE)**
• **Lang, Siegfried, Dr.**
**67071 Ludwigshafen (DE)**
• **Reich, Hans-Bernd**
**67141 Neuhofen (DE)**

(56) Entgegenhaltungen:
**EP-A- 0 258 761**   **WO-A-95/28841**
**US-A- 4 719 106**

EP 0 756 820 B1

**Beschreibung**

Die Erfindung betrifft die Verwendung von Zubereitungen, enthaltend ein Iodophor, wobei der Träger aus einer Mischung von Dextrin mit einem Poly-N-vinyllactam, nämlich Poly-N-vinyl-pyrrolidon (PVP oder Polyvidon) oder Poly-N-vinylcaprolactam oder Mischungen daraus, besteht. Weiterhin betrifft die Erfindung im wesentlichen bindemittelfreie Tabletten, Pellets oder Granulate.

Auf dem Gebiet der Feindesinfektionsmittel ist PVP-Iod ein lange etabliertes, jedoch kostenintensives Produkt. Auch die Herstellung von saccharidhaltigen Iodophoren durch Polymerisation von Vinylpyrrolidon in Anwesenheit der jeweiligen Oligo- oder Polysaccharide ist aus EP-A-526 800 bekannt. Derartige Produkte sind zwar preiswerter als PVP-Iod, aber entsprechen nicht den Anforderungen an PVP-Iod. Außerdem sind sie bisher pharmakologisch nicht akzeptiert.

EP-B-196 813 beschreibt Mischungen von PVP-Iod mit Zucker, EP-A-259 982 mit Zuckeralkoholen und gegebenenfalls Zucker, EP-A-213 717 mit Zucker und einem gelierenden Polysaccharid. Die Herstellung ist umständlich, denn es muß zunächst das PVP-Iod hergestellt und dieses anschließend mit dem (den) Zusatzstoff(en) abgemischt werden. Ein direktes Mischen aller Komponenten ist nicht möglich. Außerdem sind die Produkte für pharmazeutische Zwecke nicht geeignet, weil ihr verfügbarer Iodgehalt, der Gehalt an freiem Iod sowie der Iodverlust in der Regel außerhalb des von den Gesundheitsämtern zugelassenen Bereichs liegt.

US 4 719 106 beschreibt Mischungen aus Polydextrose-Iod und PVP-Iod. Die Herstellung von Polydextrose für diesen Einsatzbereich ist umständlich und unwirtschaftlich: Zunächst werden Polysaccharide in Glukose zerlegt, dann wird diese in Gegenwart von Sorbit und Zitronensäure wieder polykondensiert (vgl. EP 380 248).

Aufgabe der vorliegenden Erfindung war es, die Verwendung von Zubereitungen für Desinfektionszwecke auf Basis von Iodophoren zu finden, die eine gegenüber bisher bekannten Zubereitungen verbesserte Stabilität aufweisen.

Demgemäß wurde die Verwendung von Zubereitungen, welche ein Iodophor aus

a) 20 bis 71 Gew.-% Polyvinylpyrrolidon, Poly-N-vinylcaprolactam oder Mischungen davon,

b) 20 bis 71 Gew.-% Dextrin mit einem Dextroseäquivalent-Wert von 2 bis 40,

c) 6 bis 25 Gew.-% elementares Iod, und

d) 3 bis 12,5 Gew.-% Iodidionen,

enthalten, zur Grobdesinfektion von Oberflächen, zur Herstellung von Arzneimitteln zur topischen Behandlung von Hauterkrankungen sowie in Desinfektionsmitteln für den veterinärmedizinischen Bereich gefunden.
Diese Zubereitunge, die in dem gemäß Art. 54(3) EPÜ zum Stand der Technik gehörenden Dokument WO 95/28841. A beschrieben worden sind, wiesen überraschenderweise eine gegenüber bekannten Zubereitungen verbesserte Stabilität auf.

Die Dextrine sind handelsüblich und in einfacher Weise aus Stärke durch unvollständige Hydrolyse mit verdünnter Säure, durch Hitzeeinwirkung sowie durch oxidativen oder enzymatischen Abbau mit Hilfe von Amylasen zugänglich.

Durch Hydrolyse in wäßriger Phase erhältliche Stärkeabbauprodukte eines gewichtsmittleren Moleculargewichtes von 2500 bis 25000 werden im Unterschied zu den Röstdextrinen üblicherweise als verzuckerte Stärken bezeichnet und sind als solche im Handel erhältlich.

Derartige verzuckerte Stärken sind von den Röstdextrinen u.a. dadurch chemisch verschieden, daß bei einem hydrolytischen Abbau in wäßrigem Medium (üblicherweise Suspensionen oder Lösungen), der in der Regel bei Feststoffgehalten von 10 bis 30 Gew.-% sowie vorzugsweise säure- oder enzymkatalysiert vorgenommen wird, die Möglichkeit der Rekombination und Verzweigung im wesentlichen nicht gegeben ist, was sich nicht zuletzt auch in anderen Moleculargewichtsverteilungen äußert.

Die Herstellung verzuckerter Stärken ist allgemein bekannt und u.a. in Günther Tegge, Stärke und Stärkederivate, Behr's Verlag, Hamburg 1984, S. 173 und S. 220 ff sowie in der EP-A 441 197 beschrieben. Vorzugsweise handelt es sich bei den erfindungsgemäß zu verwendenden verzuckerten Stärken um solche, deren gewichtsmittleres Moleculargewicht $M_w$ im Bereich von 4000 bis 16000, besonders bevorzugt im Bereich von 6500 bis 13000 liegt.

Die erfindungsgemäß zu verwendenden verzuckerten Stärken sind normalerweise bei Raumtemperatur in Wasser vollständig löslich, wobei die Löslichkeitsgrenze in der Regel oberhalb von 50 Gew.-% liegt. Vorzugsweise sind die Lösungen bei Raumtemperatur bei 10 bis 20 Gew.-%, besonders bevorzugt bei 30 bis 40 Gew.-% klar löslich und nicht kolloidal suspendiert.

Darüber hinaus ist es besonders empfehlenswert, solche erfindungsgemäß zu verwendenden verzuckerten Stärken anzuwenden, deren Dextroseäquivalent DE 2 bis 40, vorzugsweise 10 bis 30 und besonders bevorzugt 10 bis 20 beträgt. Der DE-Wert charakterisiert das Reduktionsvermögen bezogen auf das Reduktionsvermögen von wasserfreier Dextrose und wird nach DIN 10 308, Ausgabe 5.71, des Deutschen Normenausschusses Lebensmittel und landwirtschaftliche Produkte, bestimmt (vgl. auch Günther Tegge, Stärke und Stärkederivate, Behr's Verlag, Hamburg 1984, S. 305). Ganz besonders bevorzugt sind Maltodextrine.

Als Ausgangsstärken zur Herstellung der erfindungsgemäß zu verwendenden verzuckerten Stärken sind prinzipiell alle nativen Stärken wie Getreidestärken (z.B. Mais, Weizen, Reis oder Hirse), Knollen- und Wurzelstärken (z. B. Kartoffeln, Tapiokawurzeln oder Arrowroot) oder Sagostärken geeignet.

Ein wesentlicher Vorzug der erfindungsgemäß zu verwendenden verzuckerten Stärken ist, daß es hinsichtlich ihrer Anwendung, abgesehen von der in einfachster Weise durchzuführenden partiellen Hydrolyse der Ausgangsstärke, zu ihrer Herstellung keiner weiteren chemischen Modifizierung bedarf.

In den Beispielen wurden als verzuckerte Stärken die C* PUR Produkte 01906, 01908, 01910, 01915, 01921, 01924, 01932 oder 01934 der Cerestar Deutschland GmbH, Krefeld, eingesetzt. Sie weisen im wesentlichen alle eine bimodale Molekulargewichtsverteilung auf und sind wie folgt charakterisiert:

| Typ | $M_w$ | U | Gew.-% < 1000 | DE |
|---|---|---|---|---|
| 01906 | 20080 | 10,9 | 12,2 | 2- 5 |
| 01908 | 19290 | 10,0 | 15,9 | 8-10 |
| 01910 | 10540-12640 | 8,5-9,9 | 24,7-26,4 | 11-14 |
| 01915 | 6680-8350 | 6,8-8,4 | 32,9-34,7 | 17-19 |
| 01921 | 6700 | 7,4 | 39,1 | 20-23 |
| 01924 | 4730 | 6,8 | 53.6 | 26-30 |
| 01932 | 4500 | 7,9 | 63,2 | 33-35 |
| 01934 | 3000 | 6,0 | 68,4 | 36-39 |

Bestimmungen von $M_n$ mittels Dampfdruckosmose ergaben für die bevorzugten Typen 01910 und 01915 folgende Werte:

    1560 g/mol (1910)
    980 g/mol (1915)

U =      Uneinheitlichkeit
$M_w$ =      Gewichts-Mittelwert des Molekulargewichts
$M_n$ =      zahlenmäßiger Mittelwert des Molekulargewichts
DE =      Dextrose-Äquivalent

Für die Umsetzung des Iods und Iodids mit dem Träger im festen Zustand muß der Träger in homogener Form vorliegen. Diese homogene Form kann erzielt werden durch Trocknen der gemeinsamen Lösung der Komponenten, es genügt aber auch, die pulvrigen Komponenten gemeinsam gründlich zu vermahlen. Dies kann nach üblichen Techniken, z.B. mit Kugelmühlen, Intensivmischern, Pflugscharmischer, Taumelmischern mit Kugeln usw. erfolgen. Iod und Reduktionsmittel oder Iodid kann anschließend als Feststoff oder Lösung in dem gleichen Gefäß zugemischt werden. Die Komplexbildung geschieht durch Zusatz von soviel Iod und Iodid, daß die Endmischung 6 bis 25, vorzugsweise 15 bis 20 Gew.-% Iod und pro Mol Iod ($J_2$) 1 Mol Iodid enthält, und anschließendes mehrstündiges Tempern bei 50 bis 110°C. Das Kation des Iodids ist beliebig, in der Regel Natrium oder Kalium. Das Iodid kann durch eine äquivalente Menge eines Reduktionsmittels, das Iod zu Iodid reduziert, beispielsweise Ameisensäure und ihre Salze, vorzugsweise Ammoniumformiat, Glukose, Ascorbinsäure, Malonsäure, Oxalsäure, Ammoniumoxalat, Harnstoff, Harnstoff-H2O2, PVP-H2O2, Ammoniumcarbaminat ersetzt werden, wenn die Ausgangsiodmenge entsprechend erhöht wird. Dabei ist zu berücksichtigen, daß Dextrine aufgrund ihrer aldehydischen Endgruppen auch ein gewisses Reduktionsvermögen für Iod haben.

Die Gewichtsanteile der fertigen Gesamtmischung (im Fall einer Lösung bezogen auf den Festgehalt) liegen bei 20 bis 71, vorzugsweise 30 bis 60 % PVP oder Poly-N-vinylcaprolactam, 20 bis 71, vorzugsweise 30 bis 60 % Dextrin, 9 bis 37,5 % Iod, wovon jeweils ein Drittel in Form von Iodidionen vorliegt, und 0 bis 900, vorzugsweise 0 bis 500 % Wasser.

Die polymeren Vinyllactame haben K-Werte nach H. Fikentscher (Cellulosechemie 13 (1932), 58 bis 64 und 71 bis 74) im Bereich von 12 bis 100, vorzugsweise 25 bis 70. Auch vernetztes PVP ist für die Herstellung von festem Iodophor einsetzbar und kann mit in Wasser unlöslichen Stärken zu einem Iodkomplex umgesetzt werden und so z. B. als Wundauflage Verwendung finden.

Weiterhin sind auch Mischungen von Polyvinylalkohol, Polyvinylacetat, teilweise hydrolysiertes Polyvinylacetat, Polyacrylsäure oder deren Copolymere mit Vinylpyrrolidon in Mischungen mit Maltodextrinen zum Iodkomplex umsetzbar.

Die Bestimmung des verfügbaren Iodgehaltes erfolgt nach dem Deutschen Arzneimittel Codex (DAC) 1986, 2. Ergänzung 1990, für Polyvidon-Iod. Dort ist der Gehalt zwischen mindestens 9 und höchstens 12 % verfügbarem Iod festgelegt. Gleiches gilt für die USP XXII (Povidone-Iodine). Auch die Bestimmung des Iodid-Gehaltes ist dort beschrieben. Der verfügbare Iodgehalt entspricht dem durch Titration mit Thiosulfat meßbaren Wert. Die Bestimmung des freien Iodgehalts erfolgt nach D. Horn und W. Ditter "PVP-Iod in der operativen Medizin", S. 7 ff., Springer-Verlag, Heidelberg 1984.

Auch der Gesamtgewichtsverlust von höchstens 8 % mit 0,5 g Substanz beim Trocknen im Trockenschrank bei 100 bis 105°C (Trocknungsverlust) ist im DAC 1986, 3. Lieferung 1988, festgelegt und wird von den erfindungsgemäßen Produkten eingehalten.

Der Verlust des verfügbaren Iods (Iodverlust) bei einer Wärmelagerung gibt Aufschluß über die Stabilität des Komplexes und wird wie folgt bestimmt:

Die Bestimmung erfolgt aus einer PVP-Iodlösung, die 1 % verfügbares Iod enthält. Diese wird wie folgt hergestellt: In einem 100 ml Schlifferlenmeyerkolben werden x g PVP-Iod-Probe eingewogen und mit Wasser auf ein Gesamtgewicht von 50 g ergänzt.

Berechnung der einzuwiegenden Menge x in Gramm:

$$x = \frac{5000}{(100 - TV) \cdot VJ}$$

TV =  % Trocknungsverlust nach DAC
VJ =  % Verfügbares Iod nach DAC

Die Lösung wird 3 Stunden lang geschüttelt.

Nach dem Schütteln werden 5,0 ml mit einer geeichten Vollpipette in einen 250 ml Erlenmeyerkolben pipettiert, mit ~ 100 ml dest. Wasser und 1 Tropfen Essigsäure verdünnt und bis zum Endpunkt (farblos bis schwache Gelbfärbung) möglichst rasch mit 0,02 n Natriumthiosulfatlösung titriert (Verbrauch V).

Nachdem die bisher bekannten Streckungsmittel für PVP-Iod, nämlich Zucker, Zuckeralkohole und gelbildende Polysaccharide sowie die aufwendige Polydextrose, keine befriedigende Lösung erbracht hatten, indem sie den Pharma-Zulassungsbestimmungen nicht genügen, war nicht zu erwarten, daß die technisch einfachere Lösung gemäß der Erfindung bessere Ergebnisse liefern könnte. Die Erfindung erschließt somit in überraschend einfacher Weise neue, gegenüber PVP-Iod preiswertere, technisch mindestens gleichwertige Iodophore.

Wärmelagerung

Die PVP-Iodlösung wird bis 1 cm unter dem Stopfen in eine braune 25 ml Schliffflasche gefüllt und anschließend 15 Stunden lang bei 80°C ± 0,5°C im Wärmeschrank gelagert. Etwa 15 Minuten nach Einbringen der Flasche in den Wärmeschrank wird durch Anheben des Stopfens kurz belüftet. Nach 15stündiger Lagerung werden nach Abkühlen des Flascheninhalts 5,0 ml entnommen und der Gehalt an verfügbarem Iod wie im DAC beschrieben mit 0,02 n Natriumthiosulfat-Lösung bestimmt (Verbrauch N).

Berechnung des Iodverlustes

$$\% \text{ Iodverlust} = \frac{V - N}{V} \cdot 100$$

dabei bedeuten:

V =  Verbrauch an $Na_2S_2O_3$-Lösung <u>vor</u> der Lagerung [ml]
N =  Verbrauch an $Na_2S_2O_3$-Lösung <u>nach</u> der Lagerung [ml]

Bemerkung:

Um Unregelmäßigkeiten (Temperaturschwankungen, Stromausfall etc.) während der Lagerung erkennen zu können, ist es zweckmäßig, eine Vergleichsprobe mit bekanntem Iodverlust mit zu analysieren.

Die Dextrine sind allein nicht in der Lage, ausreichend stabile Iodkomplexe zu bilden. Überraschenderweise können sie es aber in Mischung mit PVP oder Poly-N-vinylcaprolactam doch, denn gleiche Mengen der Mischung einerseits und von ungemischtem PVP oder Poly-N-vinylcaprolactam andererseits nehmen gleiche Mengen Iod auf, und zwar mit gleicher Bindungskraft. Es liegt also offenbar bei der Mischung eine Art Synergismus vor. Im Gegensatz zu Propfpolymeren handelt es sich bei den Mischungen um pharmakologisch akzeptierte Produkte. Gegenüber dem PVP-Iod bieten die erfindungsgemäßen Iodophore neben ökologischen Vorzügen, die auf der guten biologischen Abbaubarkeit des Dextrinanteils beruhen, wirtschaftliche Vorteile, da die Einsatzstoffkosten entscheidend reduziert sind.

Die erfindungsgemäßen Zubereitungen weisen eine verbesserte Stabilität und dadurch auch hinsichtlich der desinfizierenden Wirkung verbesserte Eigenschaften auf.

Die Zubereitungen können in Form von Salben, Lösungen, Shampoos, Cremes, Seifen, Gelen, Suppositorien, in Gelatinekapseln, Gurgellösungen, Sprays oder Stiften wie Lippenstiften eingesetzt werden. Bevorzugt werden dabei Formulierungen mit einem pH von 1 bis 8, bevorzugt 2 bis 7, besonders bevorzugt 3 bis 6.

Weiterhin können erfindungsgemäß Tabletten aus dextrinhaltigem Iodophor hergestellt werden. Besonders vorteilhaft ist daran, daß der Iodophor überraschenderweise, im Gegensatz zu bekannten Iodophoren, direkt tablettierbar ist, also ohne jedes weitere Tablettier-Bindemittel zu Tabletten gepreßt werden kann. Auf diese Weise können in einfacher und wirtschaftlicher Weise bindemittelfreie Iodophor-haltige Tabletten erhalten werden. Gewünschtenfalls können auch geringe Mengen an Additiven wie Schmiermittel, beispielsweise Polyethylenglykole oder Fettsäuresalze wie Magnesiumstearat, in die Tabletten eingearbeitet werden, ebenso auch Sprengmittel wie beispielsweise Crospovidon.

Besonders vorteilhaft sind Brausetabletten, enthaltend dextrinhaltigen Iodophor und Alkali- und/oder Erdalkali-Hydrogencarbonate oder Carbonate. Durch das Aufschwimmen der Tablette kann die aktive Substanz gleichmäßiger im Wasser verteilt werden, ohne daß umrühren erforderlich wäre. Dies ist besonders vorteilhaft, wenn größere Wassermengen behandelt werden sollen, beispielsweise bei der Trinkwasserbehandlung oder beim Fischfarming.

Die dextrinhaltigen Iodophore können auch zu Pellets oder Granui laten verarbeitet werden, ohne daß Bindemittel zugesetzt werden müssen.

Die Tabletten, Pellets oder Granulate können auch als Depotpräparate mit langsam löslichen Überzügen hergestellt werden.

Die Zubereitungen eignen sich vor allem zur Verwendung in der Grobdesinfektion von Oberflächen.

Weiterhin eignen sich die erfindungsgemäßen Zubereitungen auch zur Herstellung von Mitteln zur Behandlung von Hauterkrankungen wie Dekubitus, variköse Ulcera, Hautmykosen, Pyodermien, Akne, Vagitiniden sowie zur Behandlung von Brandwunden.

Weiterhin können die erfindungsgemäßen Zubereitungen für den Bereich der Veterinärmedizin verwendet werden, beispielsweise in der Gerätedesinfektion, zur Euterdesinfektion, in der Fischzucht, beispielsweise zur Desinfektion von Fischeiern, in der Stalldesinfektion, vor allem bei der Hühnerzucht, speziell im Eiablagebereich. Sie eignen sich auch zur Herstellung von Arzneimitteln zur Behandlung von Diarrhoe bei Tieren.

Gerade beim Einsatz in der Veterinärmedizin oder bei der Tieraufzucht ist es von Vorteil Tabletten, Granulate oder Pellets einzusetzen. Diese Formen sind besser dosierbar als Pulver, vermeiden Staubbelastungen, können direkt appliziert werden oder einfach mit Futter vermischt werden.

In den nachstehend verwendeten Zubereitungen wurde ein Iodophor aus 53 Gew.-% Maltodextrin CPUR 01915 (DE-Wert 17 - 19), 30 Gew.-% PVP, K-Wert 30 mit einem Gesamtjodgehalt von 17 Gew.-% (11 Gew.-% $I_2$, 6 Gew.-% Jodid) verwendet.

Bestimmung der freien Jod-Konzentration

Tabelle 1

| Verfügbares Iodgehalt [g/l] | freies Iod [ppm] |
|---|---|
| 0,1 | 18,6 |
| 1 | 15,23 |
| 5 | 6,37 |

Tabelle 1   (fortgesetzt)

| Verfügbares Iodgehalt [g/l] | freies Iod [ppm] |
|---|---|
| 10 | 4,06 |

Bestimmung der antibakteriellen Wirkung

Zur orientierenden Untersuchung wurde die minimale Hemmkonzentration (MHK) von verschiedenen Proben untersucht. Dieser Test ergibt die bakteriostatische und fungistatische Wirksamkeit der Inaktivierungssubstanz.

Geprüft wird nach den Richtlinien für die "Prüfung und Bewertung chemischer Desinfektionsverfahren (Stand: 01.01.1981)" der Deutschen Gesellschaft für hygiene und Mikrobiologie (DGHM). Die Auswertung erfolgte nach 72 stündiger Bebrütung bei 36°C. Die Verdünnung erfolgte mit Wasser standardisierter Härte ohne weitere Hilfsmittel (Tenside). Die Einstellung des pH-Wertes auf 7,2 wurde mit 0,1 mol/l HCl durchgeführt. Die Abstufung der Prüfkonzentrationen erfolgte gemäß den nachfolgend aufgeführten Verdünnungsschritten. Alle Angaben in Vol.-%.

100, 75, 50, 25, 20, 15, 10, 7,5, 5,0, 4,0, 3,0, 2,5, 2,0, 1,5, 1,00, 0,75, 0,50, 0,250, 0,10, 0,050, 0,025, 0,01250, 0,00625 (usw. in geometrischer Reihe, d.h. jeweils um Faktor 0,5 reduzierte Konzentration).

Bei den Keimen handelt es sich um Staphylococcus aureus (STA), Escherichia coli (EC), Proteus mirabilis (PRM), Pseudomonas aeruginosa (PSA) und Candida albicans (CA); somit sind gram-negative, gram-positiver Erreger und Pilze vertreten.

Tabelle 2:

| MHK-Werte gemäß DGHM | | |
|---|---|---|
| | PVP-Maltodextrin Iod | z. Vgl. PVP-Iod |
| STA | 0,75 | 1,5 |
| EC | 0,75 | 1,5 |
| PRM | 1,00 | 1,5 |
| PSA | 0,75 | 1,5 |
| CA | 0,75 | 1,0 |

Formulierungsbeispiele:

| 1.) | 10 g | PVP-Maltodextrin-Iod Komplex | 1a.) | 10 g | PVP-Iod |
|---|---|---|---|---|---|
| | 10 g | Ethanol abs. | | 10 g | Ethanol abs. |
| | 80 g | Wasser | | 80 g | Wasser |
| 2.) | 10 g | PVP-Maltodextrin-Iod Komplex | 2a.) | 10 g | PVP-Iod |
| | 20 g | Ethanol abs. | | 20 g | Ethanol abs. |
| | 70 g | Wasser | | 70 g | Wasser |
| 3.) | 10 g | PVP-Maltodextrin-Jod-Komplex | | | |
| | 30 g | Ethanol abs. | | | |
| | 60 g | Wasser | | | |
| 4.) | 10 g | PVP-Maltodextrin-Jod-Komplex | | | |
| | 10 g | Ethanol abs. | | | |
| | 0,5 g | DOR 9, Lokalanästhetikum Dodecanol/9 mol EO | | | |
| | 79,5 g | Wasser | | | |
| 5.) | 10 g | PVP-Maltodextrin-Jod-Komplex | | | |
| | 20 g | Ethanol abs. | | | |
| | 1 g | Kollidon 90 F, PVP K90 | | | |

(fortgesetzt)

| Formulierungsbeispiele: | | | | | |
|---|---|---|---|---|---|
| 0,5 g | DOR 9 | | | | |
| 68,5 g | Wasser | | | | |

| | Wert nach Herstellung | nach 15 h bei 80°C | Iodverlust |
|---|---|---|---|
| 1.) | 1,07 % | 1,01 % | 5,6 % |
| 1a.) | 1,08 % | 0,99 % | 8,3 % |
| 2.) | 1,12 % | 1,04 % | 7,1 % |
| 2a.) | 1,09 % | 1,00 % | 8,3 % |
| 3.) | 1,12 % | 1,07 % | 4,5 % |
| 4.) | 1,08 % | 1,01 % | 6,5 % |
| 5.) | 1,06 % | 0,99 % | 6,6 % |

Lagerstabilität von PVP-Maltodextrin-Iod in verschiedenen zubereitungen.

| Teat Dip | Lösung |
|---|---|
| 3,00 g PVP-Maltodextrin-Iod<br>7,00 g Glycerin<br>0,80 g Eisessig<br>3,55 g NaOH 5 %<br>85,65 g Wasser | 10,00 g PVP-Maltodextrin-Iod<br>0,25 g Cremophor NP 10[+]<br>0,50 g Lutrol E 300<br>89,25 g Pufferlösung |
| | Pufferlösung: |
| | 48,5 ml einer 0,1 molaren Zitronensäurelösung<br>51,5 ml einer 0,2 molaren $Na_2 HPO_4 * 12 H_2O$-Lösung |
| Flüssigseife | |
| 7,5 g PVP-Maltrodextrin-Iod<br>25,0 g Neutronyx S 60[*]<br>4,0 g Super Amide L 9[2]<br>63,5 g Wasser | |

[+] Nonylphenol + 10 mol EO Polyethylenglykol MG 300

[*] Ammoniumnonoxynol-4-sulfat

[2] Laurinsäurediethanolamid

| | Wert nach Herstellung | nach 14 Tagen bei: Raumtemp. |
|---|---|---|
| "Teat Dip" | | |
| Gehalt an Iod | 0,34 % | 0,33 % |
| Iodverlust | | 2,9 % |
| "Lösung" | | |
| Gehalt an Iod | 1,07 % | 1,06 % |
| Iodverlust | | 0,9 % |
| "Flüssigseife" | | |
| Gehalt an Iod | 0,79 % | 0,77 % |

(fortgesetzt)

|  | Wert nach Herstellung | nach 14 Tagen bei: Raumtemp. |
|---|---|---|
| "Flüssigseife" |  |  |
| Iodverlust |  | 2,5 % |

Lagerstabilität von PVP-Maltodextrin-Iod in verschiedenen Zubereitungen.

| Teat Dip | | Lösung | | |
|---|---|---|---|---|
| 3,00 g | PVP-Maltodextrin-Iod | pH 2,0 | 10,0 g | PVP-Maltodextrin-Iod |
| 7,00 g | Glycerin | | 90,0 g | Wasser |
| 0,80 g | Eisessig | | | |
| 3,55 g | NaOH 5 % | pH 3,0 | 10,0 g | PVP-Maltodextrin-Iod |
| 85,35 g | Wasser | | 90,0 g | Pufferlösung: |
| 0,30 g | K-Iodat | | | 71,50 ml einer 0,1 molaren Zitronensäurelösung |
| | | | | 28,50 ml einer 0,2 molaren $Na_2 HPO_4$ * 12 $H_2O$-Lösung |
| | | pH 4,5 | 10,0 g | PVP-Maltodextrin-Iod |
| | Flüssigseife | | 90,0 g | Pufferlösung: |
| 7,5 g | PVP-Maltodextrin-Iod | | | 48,5 ml 0,1 mol Zitronensäure |
| 25,0 g | Neutronyx S 60 | | | 51,5 ml 0,2 mol Na2 $HPO_4$ * 12 H2O |
| 4,0 g | Super Amide L 9 | | | |
| 63,2 g | Wasser | pH 4,5 | 10,0 g | PVP-Maltodextrin |
| 0,30 g | K-Iodat | | 90,0 g | Pufferlösung: 36,85 ml 0,1 mol Zitronensäure |
| | | | | 63,15 ml 0,2 mol $Na_2$ HPO4 * 12 H2O |

| | Wert nach Herstellung ohne/mit Iodat 0,3 g | nach 14 Tagen bei : ohne/mit Iodat 0,3 g Raumtemp. | 52°C |
|---|---|---|---|
| **Teat Dip** | | | |
| Gehalt an Iod | 0,41 % | 0,40 % | 0,42 % |
| Iodverlust | | 2,4 % | 0,0 % |
| pH-Wert | 4,28 | 4,24 | 4,10 |
| **Flüssigseife** | | | |
| Gehalt an Iod | 0,87 % | 0,89 % | 0,86 % |
| Iodverlust | | 0,0 % | 1,1 % |
| pH-Wert | 5,13 | 5,00 | 3,78 |
| **Lösung pH 2** | | | |
| Gehalt an Iod | 1,10 % | 1,07 % | 1,03 % |
| Iodverlust | | 2,7 % | 6,4 % |
| pH-Wert | 1,60 | 1,58 | 1,40 |
| **Lösung pH 3** | | | |
| Gehalt an Iod | 1,10 %/1,56 % | 1,11 %/1,44 % | |
| Iodverlust | | 0,9 %/7,7 % | |
| pH-Wert | 2,77/3,14 | 2,75/3,21 | |
| **Lösung pH 4,5** | | | |
| Gehalt an Iod | 1,12 %/1,28 % | 1,08 %/1,29 % | |

(fortgesetzt)

| | Wert nach Herstellung ohne/mit Iodat 0,3 g | nach 14 Tagen bei : ohne/mit Iodat 0,3 g Raumtemp. | 52°C |
|---|---|---|---|
| Lösung pH 4,5 | | | |
| Iodverlust | | 3,6 %/0,0 % | |
| pH-Wert | 4,25/4,63 | 4,18/4,65 | |
| Lösung pH 5,5 | | | |
| Gehalt an Iod | 1,09 %/1,13 % | 1,00 %/1,19 % | |
| Iodverlust | | 8,3 %/0,0 % | |
| pH-Wert | 5,43/5,40 | 5,21/4,46 | |

Tabletten

Zur Herstellung der Tabletten wurde eine Tablettenpresse der Fa. Kilian + Co. GmbH, Köln, Type KS verwendet. Die Oberstempel-Einstellung betrug 4 - 5, die Unterstempel-Fülltiefe 10 mm bei einem Durchmesser von 12 mm, bei 30 Hüben/min.

Zusammensetzung der Tablettiermischungen:

| T1 | 100 Gew.-% | eines Iodophors aus PVP-Maltodextrin-Jod der eingangs geschilderten Zusammensetzung |
|---|---|---|
| T2 | 99 Gew.-% | PVP-Maltodextrin-Jod |
| | 1 Gew.-% | Polyethylenglykol 6000 |
| T3 | 98 Gew.-% | PVP-Maltodextrin-Jod |
| | 1 Gew.-% | Polyethylenglykol 6000 |
| | 1 Gew.-% | Crospovidon |

**Patentansprüche**

**1.** Verwendung von Zubereitungen, enthaltend ein Iodophor aus

a) 20 bis 71 Gew.-% Polyvinylpyrrolidon, Poly-N-vinylcaprolactam oder Mischungen daraus,

b) 20 bis 71 Gew.-% Dextrin mit einem Dextroseäquivalent-Wert von 2 bis 40,

c) 6 bis 25 Gew.-% elementares Iod, und

d) 3 bis 12,5 Gew.-% Iodidionen,

zur Grobdesinfektion von Oberflächen.

**2.** Verwendung von Zubereitungen, enthaltend ein Iodophor aus

a) 20 bis 71 Gew.-% Polyvinylpyrrolidon, Poly-N-vinylcaprolactam oder Mischungen daraus,

b) 20 bis 71 Gew.-% Dextrin mit einem Dextroseäquivalent-Wert von 2 bis 40,

c) 6 bis 25 Gew.-% elementares Iod, und

d) 3 bis 12,5 Gew.-% Iodidionen,

zur Herstellung von Arzneimitteln zur topischen Behandlung von Hauterkrankungen.

3. Verwendung von Zubereitungen, enthaltend ein Iodophor aus

    a) 20 bis 71 Gew.-% Polyvinylpyrrolidon, Poly-N-vinylcaprolactam oder Mischungen daraus,

    b) 20 bis 71 Gew.-% Dextrin mit einem Dextroseäquivalent-Wert von 2 bis 40,

    c) 6 bis 25 Gew.-% elementares Iod, und

    d) 3 bis 12,5 Gew.-% Iodidionen,

in Desinfektionsmitteln für den veterinärmedizinischen Bereich.

4. Im wesentlichen bindemittelfreie Tabletten, Pellets oder Granulate, hergestellt aus einem pulverförmigen Iodophor aus

    a) 20 bis 71 Gew.-% Polyvinylpyrrolidon, Poly-N-vinylcaprolactam oder Mischungen daraus,

    b) 20 bis 71 Gew.-% Dextrin mit einem Dextroseäquivalent-Wert von 2 bis 40,

    c) 6 bis 25 Gew.-% elementares Iod, und

    d) 3 bis 12,5 Gew.-% Iodidionen.

**Claims**

1. The use of formulations for disinfecting surfaces.

2. The use of formulations comprising an iodophore comprising

    a) 20 - 71% by weight of polyvinylpyrrolidone, poly-N-vinylcaprolactam or mixtures thereof,

    b) 20 - 71% by weight of dextrin with a dextrose equivalent value of 2 to 40,

    c) 6 - 25% by weight of elemental iodine, and

    d) 3 - 12.5% by weight of iodide ions,

for producing pharmaceutical compositions for the topical treatment of skin disorders.

3. The use of formulations comprising an iodophore comprising

    a) 20 - 71% by weight of polyvinylpyrrolidone, poly-N-vinylcaprolactam or mixtures thereof,

    b) 20 - 71% by weight of dextrin with a dextrose equivalent value of 2 to 40,

    c) 6 - 25% by weight of elemental iodine, and

    d) 3 - 12.5% by weight of iodide ions,

in disinfectants for use in veterinary medicine.

4. Tablets, pellets or granules which are essentially binder-free and are produced from an iodophore which is in powder form and comprises

    a) 20 - 71% by weight of polyvinylpyrrolidone, poly-N-vinylcaprolactam or mixtures thereof,

b) 20 - 71% by weight of dextrin with a dextrose equivalent value of 2 to 40,

c) 6 - 25% by weight of elemental iodine, and

d) 3 - 12.5% by weight of iodide ions.

**Revendications**

1.  Utilisation de compositions contenant un iodophore constitué lui-même de

    a) 20 à 71 % en poids de polyvinylpyrrolidone, de poly-N-vinylcaprolactame ou leurs mélanges,
    b) 20 à 71 % en poids d'une dextrine présentant un équivalent de dextrose de 2 à 40,
    c) 6 à 25 % en poids d'iode élémentaire et
    d) 3 à 12,5 % en poids d'ions iodure,

    pour la désinfection grossière de surfaces.

2.  Utilisation de compositions contenant un iodophore constitué lui-même de

    a) 20 à 71 % en poids de polyvinylpyrrolidone, de poly-N-vinylcaprolactame ou leurs mélanges,
    b) 20 à 71 % en poids d'une dextrine présentant un équivalent de dextrose de 2 à 40,
    c) 6 à 25 % en poids d'iode élémentaire et
    d) 3 à 12,5 % en poids d'ions iodure,

    pour la préparation de médicaments pour le traitement topique de maladies de la peau.

3.  Utilisation de compositions contenant un iodophore constitué lui-même de

    a) 20 à 71 % en poids de polyvinylpyrrolidone, de poly-N-vinylcaprolactame ou leurs mélanges,
    b) 20 à 71 % en poids d'une dextrine présentant un équivalent de dextrose de 2 à 40,
    c) 6 à 25 % en poids d'iode élémentaire et
    d) 3 à 12,5 % en poids d'ions iodure,

    dans des produits désinfectants pour la médecine vétérinaire.

4.  Comprimés, pellets ou granulés essentiellement exempts de liants, préparés à partir d'un iodophore pulvérulent consistant lui-même en

    a) 20 à 71 % en poids de polyvinylpyrrolidone, de poly-N-vinylcaprolactame ou leurs mélanges,
    b) 20 à 71 % en poids d'une dextrine présentant un équivalent de dextrose de 2 à 40,
    c) 6 à 25 % en poids d'iode élémentaire et
    d) 3 à 12,5 % en poids d'ions iodure.